(19) Europäisches Patentamt
European Patent Office
Office européen des brevets

(11) EP 0 594 223 B1

(12) **EUROPEAN PATENT SPECIFICATION**

(45) Date of publication and mention of the grant of the patent: **01.03.2000 Bulletin 2000/09**

(51) Int. Cl.[7]: **C07H 19/073**, A61K 31/70

(21) Application number: **93120947.2**

(22) Date of filing: **15.09.1986**

# (54) Combination of therapeutic nucleosides and further therapeutic agents.

Kombination therapeutische Nukleoside mit weiteren therapeutisch wirksamen Komponenten.

Combination de nucléosides thérapeutiques et d'autre agents thérapeutiques.

(84) Designated Contracting States:
**AT BE CH DE FR GB IT LI LU NL SE**

(30) Priority: **17.09.1985 US 776899**
**27.09.1985 GB 8523878**
**12.02.1986 GB 8603447**
**14.02.1986 GB 8603719**
**04.04.1986 GB 8608272**
**23.06.1986 GB 8615322**
**23.06.1986 US 877796**
**23.06.1986 US 877284**

(43) Date of publication of application:
**27.04.1994 Bulletin 1994/17**

(62) Document number(s) of the earlier application(s) in accordance with Art. 76 EPC:
**86307071.0 / 0 217 580**

(73) Proprietor:
**THE WELLCOME FOUNDATION LIMITED**
**Greenford, Middlesex UB6 0NN (GB)**

(72) Inventors:
- **Rideout, Janet Litster**
  **Raleigh, North Carolina 27607 (US)**
- **Barry, David Walter**
  **Chapel Hill, North Carolina 27514 (US)**
- **Lehrman, Sandra Nusinoff**
  **Durham, North Carolina 27707 (US)**
- **St. Clair, Martha Heider**
  **Durham, North Carolina 27704 (US)**
- **Furman, Phillip Allen**
  **Durham, North Carolina 27713 (US)**
- **Freeman, George Andrew**
  **Cary, North Carolina 27511 (US)**
- **Zimmerman, Thomas Paul**
  **Raleigh, North Carolina 27612 (US)**
- **Wolberg, Gerlad**
  **Cary, North Carolina 27511 (US)**
- **de Miranda, Paul M.**
  **Raleigh, North Carolina 27609 (US)**
- **Shaver, Sammy Ray**
  **Chapel Hill, North Carolina 27514 (US)**
- **White, Geoffrey Dr.**
  **Chearsley Aylesbury HP18ODH (GB)**
- **Kirk III, Leone Edward**
  **Raleigh, North Carolina 27608 (US)**
- **King, Dannie Hilleary**
  **Raleigh, North Carolina 27612 (US)**
- **Clemons, Richard Harlon**
  **Cary, North Carolina 27511 (US)**

(74) Representative:
**Garrett, Michael et al**
**Glaxo Wellcome plc,**
**Glaxo Wellcome House,**
**Berkeley Avenue**
**Greenford, Middlesex UB6 0NN (GB)**

(56) References cited:
- **JOURNAL OF MEDICINAL CHEMISTRY vol. 26, no. 12 , 1983 , WASHINGTON US pages 1691 - 1696 T-S. LIN ET AL. 'Synthesis and Biological Activity of Various 3'-Azido and 3'-Amino Analogues of 5-Substituted Pyrimidine Deoxyribonucleosides.'**
- **EUROPEAN JOURNAL OF MEDICINAL CHEMISTRY vol. 20, no. 4 , 1985 pages 295 - 301 L.COLLA ET AL. 'Synthesis and Biological Activity of 3'-Azido- and 3'-Amino Substituted Nucleoside Analogs.'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **PROCEEDINGS OF THE NATIONAL ACADEMY OF SCIENCE U.S.A. vol. 71, no. 12 , 1974 , US pages 4980 - 4985 W.OSTERTAG ET AL. 'Induction of Endogeneous Virus and of Thymidine Kinase by Bromodeoxyuridine in Cell Cultures Transformed by Friend Virus.'**
- **EXPERIMENTAL CELL RESEARCH vol. 116, no. 1 , 1978 , SE pages 21 - 29 C.J.KRIEG ET AL. 'Increase in Intracisternal A-type Particles in Friend Cells During Inhibition of Friend Virus (SFFV) Release by Interferon or Azidothymidine.'**

## Description

The present invention relates to 3'-azido-nucleosides, pharmaceutically acceptable derivatives thereof, combinations containing them, and their use in therapy, particularly for the treatment or prophylaxis of certain viral and bacterial infections.

In the field of antiviral chemotherapy, few drugs exist which effectively control the virus per se, owing to the difficulty of attacking the virus while leaving uninfected host cells unimpared. It was long supposed that, with the extreme parasitic nature of viruses, all the necessary facilities for viral replication were provided by the host cell. It has recently been established that certain stages in the virus life-cycle, which vary from species to species, are specified by the virus itself, and these stages may prove susceptible to attack where they differ sufficiently from any corresponding host-cell function. However, owing to great similarity between viral and host functions, effective treatments have proven very difficult to identify.

For this reason, compounds identified as being suitable for the treatment of viral infections usually have some toxicity for the host. Thus, the ideal medication is non-toxic at antivirally effective concentrations but, in the absence of such a treatment, the compound should possess a good therapeutic ratio, that is, the concentrations at which the treatment is toxic are significantly higher than those at which an antiviral activity is observed.

One group of viruses which has recently assumed a particular importance are the retroviruses. Retroviruses form a sub-group of RNA viruses which, in order to replicate, must first 'reverse transcribe' the RNA of their genome into DNA ('transcription' conventionally describes the synthesis of RNA from DNA). Once in the form of DNA, the viral genome may be incorporated into the host cell genome, allowing it to take full advantage of the host cell's transcription/translation machinety for the purposes of replication. Once incorporated, the viral DNA is virtually indistinguishable from the host's DNA and, in this state, the virus may persist for as long as the cell lives. As it is virtually invulnerable to attack in this form, any treatment must be directed at another stage of the virus life-cycle and will, of necessity, have to be continued until all virus-infected cells have died.

HTLV-I and HTLV-II are both retroviruses and are known to be causative agents of leukaemia in man. HTLV-I infections are especially widespread and are responsible for many deaths world-wide each year.

A species of retrovirus has also been reproducibly isolated from patients with AIDS. While it has been extensively characterised, there is still some dispute as to an internationally agreeable name for the virus. It is currently known either as human T-cell lymphotropic virus III (HTLV III), AIDS associated retrovirus (ARV), or lymphadenopathy associated virus (LAV) but it is anticipated that the name to be agreed on internationally will be human immunodeficiency virus (HIV). This virus (referred to herein as HIV) has been shown preferentially to infect and destroy T-cells bearing the OKT4 surface marker and is now generally accepted as the aetiologic agent of AIDS. The patient progressively loses this set of T-cells, upsetting the overall balance of the immune system, reducing his ability to combat other infections, and predisposing him to opportunistic infections which frequently prove fatal. Thus, the usual cause of death in AIDS victims is by opportunisitc infection, such as pneumonia or cancers which may be virally induced, and not necessarily as a direct result of HIV infection. Other conditions associated with HIV infection include thrombocytopaenia purpura and Kaposi's sarcoma.

Recently, HIV has also been recovered from other tissue types, including B-cells expressing the T4 marker, macrophages and non-blood associated tissue in the central nervous system. This infection of the central nervous system is not necessarily associated with classical AIDS and has been found in patients with asymptomatic HIV infections. HIV infection of the CNS is associated with progressive demyelination, leading to wasting and such symptoms as encephalopathy, progressive dysarthria, ataxia and disorientation. Further conditions associated with HIV infection are the asymptomatic carrier state, progressive generalised lymphadenopathy (PGL) and AIDS-related complex (ARC).

It is now considered that certain, chronic, neurological infections are caused by retroviruses. Such infections include multiple sclerosis in man, for example, and caprine arthritis of encephalitis virus infections in goats, and visnamaedi infections in sheep.

Reports have described the testing of compounds against various retroviruses, for example, Friend Leukaemia Virus (FLV), a murine retrovirus. For instance Krieg et al (Exp.Cell Res., 116, (1978) 21-29) found that 3'-azido-3'-deoxythymidine affected the release of C-type particles and increased the formation of A-type particles of a FLV complex in vitro experiments, and Ostertag et al (Proc.Nat.Acad.Sci. (1974) 71, 4980-85) stated that, on the basis of antiviral activity related to the above FLV complex and a lack of cellular toxicity, 3'-azido-3'-dideoxythymidine "might favourable replace bromodeoxyuridine for medical treatment of diseases caused by DNA viruses". However, De Clerq et al (Biochem.Pharm. (1980) 29, 1849-1851) established, six years later, that 3'-azido-3'-dideoxythymidine had no appreciable activity against any viruses used in their tests, including such DNA viruses as vaccinia, HSVI and varicella zoster virus (VZV).

The compound 3'-azido-3'-deoxythymidine has particularly good activity against the following viruses: human T-cell lymphotropic virus (HTLV), especially HTLV-1, HTLV-II and HTLV-III (HIV); feline leukaemia virus, equine infectious anaemia virus, caprine arthritis virus and other lentiviruses, as well as other human viruses such as hepatitis B

virus, Epstein-Barr virus (EBV) and the causative agent of multiple sclerosis (MS). The above compound has also been found to be effective in the treatment of Kaposi's sarcoma (KS) and thrombocytopaenia purpura (TP).

It has been discovered that the above 3'-azidonucleoside cooperates synergistically with a wide range of other therapeutic agents, thereby disproportionately enhancing the therapeutic potential of both agents. Significantly less of each compound is required for treatment, the therapeutic ratio is raised and, accordingly, the risk of toxiciy from either compound is reduced.

Therefore, according to a further aspect of the present invention, there is provided a therapeutic combination of 3'-azido-3'-deoxythymidine or a pharmaceutically acceptable derivative thereof and at least one further therapeutic agent.

In particular, there is further provided 3'-azido-3'-deoxythymidine, or a pharmaceutically acceptable derivative thereof, for use in combination therapy with at least one further therapeutic agent wherein the two active agents are present in a potentiating ratio.

A "potentiating ratio" is that ratio of 3'-azido-3'-deoxythymidine or a pharmaceutically acceptable derivative thereof, to the second therapetuic agent which provides a therapeutic effect greater than the sum of the therapeutic effects of the individual components.

It will be appreciated that, while there will usually be an optimum ratio to ensure maximum potentiation, even a vanishingly small quantity of one agent will suffice to potentiate the effect of the other to some degree, and so any ratio of two potentiating drugs will still possess the required synergistic effect. However, greatest synergy is observed when the two agents are present in a ratio of 500:1 to 1:500, preferably 100:1 to 1:100, particularly 20:1 to 1:20 and especially 10:1 to 1:10.

The combinations described above are referred to herein as combinations according to the invention.

Thus, there is further provided the combinations according to the invention in the manufacture of a medicament for the treatment or prophylaxis of any of the infections or indications mentioned above.

The combinations according to the invention may conveniently be administered together, for example, in a unitary pharmaceutical formulation, or separately, for example as a combination of tablets and injections administered at the same time or different times, in order to achieve the required therapeutic effect.

In antiviral tests, it has been found that, for example, 3'-azido-3'-deoxythymidine potentiated by such diverse agents as interferons, nucleoside transport inhibitors, glucuronidation inhibitors, renal excretion inhibitors and even other therapeutic nucleosides which may not necessarily have activity against the same organisms as the first compound.

Particularly preferred types of interferon are α, β and γ, while nucleoside transport inhibitors include such agents as dilazep, dipyridamole, 6-[(4-nitrobenzoyl)thio]-9-(β-D-ribofuranosyl) purine, papavarine, mioflazine, hexobendine, lidoflazine and their acid addition salts.

Probenecid is particularly useful in combination with 3'-azido-3'-deoxythymidine as it possesses both renal excretion inhibiting activity and glucuronidation blocking activity. Examples of other compounds useful in this aspect include acetaminophen, aspirin, lorazepam, cimetidine, ranitidine, zomepirac, clofibrate, indomethacin, ketoprofen, naproxen and other compounds which compete for glucuronidation or otherwise undergo significant glucuronidation.

3'-azido-3'-deoxythymidine and its pharmaceutically acceptable derivatives are potentiated by other therapeutic nucleoside derivatives as described above, including acyclic nucleosides of the type described for example in UK patent specification No. 1 523 865, US patent speciifcation No. 4 360 522, European patent specifications Nos. 74 306, 55 239 and 146 516, and European patent applications 434 393 and Nos. 434 395, and particularly include those compounds of the general formula

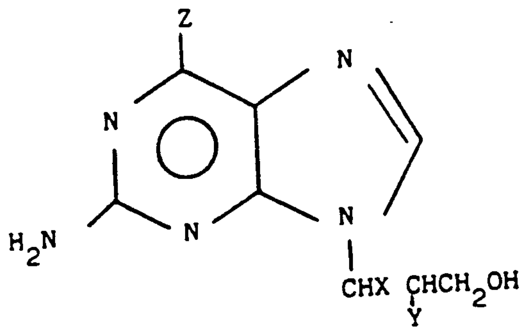

(A)

wherein Z represents a hydrogen atom or a hydroxy or amino group;
X represents

(a) an oxygen or sulphur atom or a methylene group and Y represents a hydrogen atom or a hydroxymethylene group; or

(b) methyleneoxy group ($-OCH_2$) and Y represents a hydroxy group;

and pharmaceutically acceptable derivatives thereof.

Examples of the above-mentioned derivatives are salts and esters and include base salts e.g. alkali metal (eg. sodium) or alkaline earth metal salts and pharmaceutically acceptable salts of organic acids such as lactic, acetic, malic or p-toluenesulphonic acid, as well as pharmaceutically acceptable salts of mineral acids such as hydrochloric or sulphuric acid.

Esters of the compounds of formula (A) which may be conveniently used in accordance with the present invention include those containing a formyloxy or $C_{1-16}$ (for example $C_{1-6}$)alkanoyloxy (e.g. acetoxy or propionyloxy), optionally substituted aralkanoyloxy (e.g. phenyl-$C_{1-4}$alkanoyloxy such as phenyl-acetoxy) or optionally substituted aroyloxy (e.g. benzoyloxy or naphthoyloxy) ester grouping at one or both of the terminal positions of the 9-side chain of the compounds of formula (A). The above-mentioned aralkanoyloxy and aroyloxy ester groups may be substitued, for example, by one or more halogen (e.g. chlorine or bromine) atoms or amino, nitrile or sulphamido groups, the aryl moiety of the grouping advantageously containing 6 to 10 carbon atoms.

Particularly preferred examples of compounds of the above general formula (A) for use in accordance with the present invention include 9-[(2-hydroxy-1-hydroxymethylethoxy)methyl]guanine, 2-amino-9-(2- hydroxyethoxymethyl)purine and particularly 9-(2-hydroxyethoxymethyl) guanine (acyclovir). The latter compound has been found to have a particularly good potentiating effect.

In the antibacterial field, it has also been found that a broad spectrum of antibiotics is effective in potentiating the activity of 3'-azido-3'-deoxythymidine. These include diverse agents such as: benzylpyrimidines e.g. 2,4-diamino-5-(3',4',5'-trimethoxybenzyl) pyrimidine (trimethoprim) and analogues thereof, for example as described in UK patent specification No. 1 405 246; sulphonamides e.g. sulfadimidine; rifampicin; tobramycin; fusidic acid; chloramphenicol; clindamycin and erythromycin.

Thus, in a further aspect there is provided combinations according to the invention wherein the second agent is at least one of the above-mentioned antiviral or antibacterial agents or classes of agent.

Other combinations suitable for use according to the present invention include those wherein the second agent is, for example, interleukin II, suramin, phosphonoformate, HPA 23, 2',3'-dideoxynucleosides, for example, 2',3'-dideoxycytidine and 2',3'-dideoxyadenosine, or medications such as levamisol or thymosin to increase lymphocyte numbers and/or function as appropriate.

It will further be appreciate that the compounds and combinations according to the invention may also be used in conjunction with other immune modulating therapy including, for example, bone marrow and lymphocyte transplants.

Certain of the retroviral infections described above, for example AIDS, are commonly associated with opportunistic infections. Thus it will be appreciated that, for example, a combination of 3'-azido-3'-deoxythymidine (AZT) and acyclovir would prove particularly useful in the treatment of an AIDS patient with an opportunistic herpes infection, while a combination of AZT and 9-[(2-hydroxy-1-hydroxy methylethoxy)methyl]guanine would be useful in the treatment of an AIDS patient with an opportunistic cytomegalovirus infection.

By "a pharmaceutically acceptable derivative" is meant any pharmaceutically acceptable salt, ester or salt of such ester, or any other compound which upon administration to the recipient, is capable of providing (directly or indirectly) the parent 3'-azidonucleoside or a therapeutically effective metabolite or residue thereof. An example of a non-ester compound is the derivative wherein the 5'-C and 2-C-atoms are linked by an oxygen atom to form an anhydro group.

Preferred esters include carboxylic acid esters in which the non-carbonyl moiety of the ester grouping is selected from straight or branched chain alkyl, alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); and mono-, di-or triphosphate esters. Any reference to any of the above compounds also includes a reference to a pharmaceutically acceptable salt thereof.

With regard to the above-described esters, unless otherwise specified, any alkyl moiety present advantageously containins 1 to 18 carbon atoms, particularly 1 to 4 carbon atoms. Any aryl moiety present in such esters advantegously comprises a phenyl group.

Examples of pharmaceutically acceptable salts and pharmaceutically acceptable derivatives thereof include base salts, e.g. derived from an appropriate base, such as alkali metal (e.g. sodium), alkaline earth metal (e.g. magnesium) salts, ammonium and $NX^+_4$ (wherein X is $C_{1-4}$ alkyl) and mineral acid salts, such as the hydrochloride.

Specific examples of pharmaceutically acceptable derivatives of 3'-azido-3'-deoxythymidine that may be used in accordance with the present invention include the monosodium salt and the following 5' esters: monophosphate; disodium monophosphate; diphosphate; triphosphate; acetate; 3-methyl-butyrate; octanoate; palmitate; 3-chloro benzoate; benzoate; 4-methyl benzoate; hydrogen succinate; pivalate; and mesylate.

3'-Azido-3'-deoxythymidine and its pharmaceutically acceptable derivatives, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

In general a suitable dose will be in the range of 3.0 to 120 mg per kilogram body weight of the recipient per day, preferably in the range of 6 to 90 mg per kilogram body weight per day, and most preferably in the range 15 to 60 mg per kilogram body weight per day. The desired dose is preferably presented as two, three, four, five, six or more sub-doses administered at appropriate intervals throughtout the day. These sub-doses may be administerd in unit dosage forms, for example, containing 10 to 1500 my, preferably 20 to 1000 mg, and most preferably 50 to 700 mg of active ingredient per unit dosage form.

Experiments with 3'-azido-3'-deoxythymidine suggest that a dose should be administered to achieve peak plasma concentrations of the active compound of from about 1 to 75 μM, preferably about 2 to 50 μM, most preferably about 3 to about 30 μM. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

The combinations according to the invention may be administered in a similar manner to that described above to provide the desired therapeutic dosages of the active ingredient and the further therapeutic agent concerned. The dosage of the combination will depend on the condition being treated, the particular active ingredient and the further therapeutic agent concerned and other clinical factors such as the weight and condition of the patient and the route of administration of the combination. As indicated above, the active ingredient and the further therapeutic agent may be administered simultaneously (e.g. in a unitary pharmaceutical formulation) or separately (e.g. in separate pharmaceutical formulations) and, in general, the combinations may be administered by the topical, oral, rectal or parenteral (e.g. intravenous, subcutaneous or intramuscular) routes.

In particular, combinations according to the invention wherein the second agent is a nucleoside transport inhibitor, may be administered to the subject concerned in conventional manner. However, for administration by the oral route a dosage of the active ingredient of 1 to 200 mg/kg/day, preferably 5 to 50 mg/kg/day, is generally sufficient. The amount of nucleoside transport inhibitor in the combination is independent of the amount of active ingredient specified above and is sufficient to inhibit nucleoside transport effectively and is preferably in the range of 0.1 to 100 mg/kg/day, and particularly in the range 1 to 20 mg/kg/day.

The combinations according to the invention, wherein the second agent is either a renal excretion inhibitor or a glucuronidation inhibitor or both, may be administered to the subject concerned in conventional manner. However, for administration by the oral routes dosage of active ingredient of 1 to 200 mg/kg/day, preferably 5 to 50 mg/kg/day, is generally sufficient. For administration by the parenteral route, a dosage of active ingredient of 1 to 100 mg/kg/day, preferably 2 to 30 mg/kg/day is generally sufficient. The amount of the renal excretion / glucuronidation inhibitor in the combination is independent of the amount of active ingredient and is sufficient in the range of 4-100 mg/kg/day, preferably 5-60 mg/kg/day, most preferably 10-40 mg/kg/day.

The combinations according to the invention, wherein the second agent is an interferon, beneficially comprise active ingredient at from 5 to 250 mg per kg per day; and the suitable effective dose range of interferon is $3x10^6$ to $10x10^6$ IU per square meter of body surface per day, preferably $4x10^6$ to $6x10^6$ IU per square meter per day. The active ingredient and interferon should be administered in the ratio of from about 5 mg per kg of active ingredient to $3x10^6$ IU per square meter of interferon to about 250 mg per kg per day of active ingredient to $10x10^6$ IU per square meter per day of interferon, preferably from about 5 mg per kg per day of active ingredient to $4x10^6$ IU per square meter per day of interferon to about 100 mg per kg per day of active ingredient to $6x10^6$ IU per square meter per day of interferon.

Interferon is preferably administered by injection (s.c., i.m. or i.v., for example), while the active ingredient is preferably administered orally or by injection, but may be administered by any mode described herein. Interferon and active ingredient may be administered together or separately, and the daily dose of each may preferably be administered as divided doses.

For combinations according to the invention wherein the second agent is another therapeutic nucleoside, a suitable effective oral dose range of active ingredient is 2.5 to 50 mg per kg of body weight per day, preferably 5 to 10 mg per kg per day; and the suitable effective oral dose range of the antibacterial agent is in the range of 2 to 1000 mg/kg/day, preferably 50 to 500 mg/kg/day. The preferred ratios of active ingredient to antibacterial agent are in the range of 20:1 to 1:500, particularly 2:1 to 1:125.

It will be appreciated that, while combinations containing three or more therapeutic agents are not specifically described herein, such combinations do form a part of the present invention. For example, a combination of 3'-azido-3'-deoxythymidine (AZT) with acyclovir and probenecid has the double advantage of both potentiating the activity of AZT and increasing its availability. Likewise, combinations of 3'-azido-3'-deoxythymidine with two or more of the same variety

of second therapeutic agent also form part of the invention, for example, AZT with sulfadimidine and trimethoprim.

While it is possible for the active ingredient to be administerd alone it is preferable to present it as a pharmaceutical formulation. The formulations of the present invention comprise at least one active ingredient, as above defined, together with one or more acceptable carriers thereof and optionally other therapeutic agents. Each carrier must be "acceptable" in the sens of being compatible with the other ingredients of the formnulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

3'-Azido-3'-deoxythymidine and its pharmaceutically acceptable derivatives, may be prepared in conventional manner using techniques that are well known in the art, e.g. as described in: Synthetic Procedures in Nucleic Acid Chemistry (1, 321 (1968), T.A.Krenitsky et al), J.Med.Chem. (26, 981 (1983)); Nucleic Acid Chemistry, Improved and New Synthetic Processes, Methods and Techniques (Parts 1 and 2, Ed. L.D.Townsend, R.S.Tipson, (J.Wiley) 1978); J.R.Horwitz et al. (J.Org.Chem. 29, (July 1964) 2076-78); M.Imazawa et al, (J.Org.Chem, 43, (15) (1978) 3044-3048); K.A.Watanabe et al. (J.Org.Chem., 45, 3274 (1980)); and R.P.Glinsky et al. (J.Chem.Soc.Commun., 915, (1970)), which disclosures are herein incorporated by reference, or using techniques that are analogous to those described therein.

The following Examples are intended for illustration only and are not intended to limit the scope of the invention in any way. The term 'Active Ingredient' as used in the Examples means 3'-azido-3'-deoxy thymidine or a pharmaceutically acceptable derivative thereof.

Example 1: Tablet Formulations

The following formulations A and B are prepared by wet granulation of the ingredients with a solution of povidone, followed by addition of magnesium stearate and compression. Formulation A

| | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 500 | 300 |

Formulation B

| | mg/tablet | mg/tablet |
|---|---|---|
| (a) Active ingredient | 250 | 250 |
| (b) Lactose 150 | - | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

Formulation C

| | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose used in formulation E is of the direct compression type (Dairy Crest- "Zeparox").

Formulation D

| | mg/capsule |
|---|---|
| Active Ingredient | 250 |
| Pregelatinised Starch NF15 | 150 |
| | 400 |

Formulation E

| | mg/capsule |
|---|---|
| Active ingredient | 250 |
| Lactose | 150 |
| Avicel | 100 |
| | 500 |

Formulation F (Controlled Release Formulation)

The formulation is prepared by wet granulation of the incredients (below) with a solution of povidone followed by the addition of magnesium stearate and compression.

| | mg/tablet |
|---|---|
| (a) Active Ingredient | 500 |
| (b) Hydroxypropylmethylcellulose (Methocel K4M Premium) | 112 |
| (c) Lactose B.P. | 53 |
| (d) Povidone B.P.C. | 28 |
| (e) Magnesium Stearate | 7 |
| | 700 |

Example 2: Capsule Formulations

Formulation A

A capsule is prepared by admixing the ingredients of Formulation D in Example 1 above and filling into a two-part hard gelating capsule. Formulation B (infra) is prepared in a similar manner.

Formulation B

| | mg/capsule |
|---|---|
| (a) Active ingredient | 250 |
| (b) Lactose B.P. | 150 |
| (c) Sodium Starch Glycollate | 25 |
| (d) Magnesium Stearate | 2 |
| | 420 |

Formulation C

| | mg/capsule |
|---|---|
| (a) Active ingredient | 250 |
| (b) Macrogol 4000 BP | 350 |
| | 600 |

Capsules are prepared by melting the Macrogol 4000 BP, dispersing the active ingredient in the melt and filling the melt into a two-part hard gelatin capsule.

Formulation D

| | mg/capsule |
|---|---|
| Active ingredient | 250 |
| Lecithin | 100 |
| Arachis Oil | 100 |
| | 450 |

Capsules are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients a, b and c using an extruder, followed by spheronisation of the extrudate and drying. The dried pellets are then coated with release-controlling membrane (d) and filled into a two-piece, hard gelatin capsule.

| | mg/capsule |
|---|---|
| (a) Active Ingredient | 250 |
| (b) Microcrystalline Cellulose | 125 |
| (c) Lactose B.P. | 125 |
| (d) Ethyl Cellulose | 13 |
| | 513 |

Example 3: Injectable Formulation

Formulation A

| | | |
|---|---|---|
| Active Ingredient | | 0.200g |
| Hydrochloric acid solution, 0.1M | q.s. to pH | 4.0 to 7.0 |
| Sodium hydroxide solution, 0.1M | q.s. to pH | 4.0 to 7.0 |
| Sterile water | q.s. to | 10ml |

The active ingredient is dissolved in most of the water (35°-40°C) and the pH adjusted to between 4.0 and 7.0 with the hydrochloric acid or the sodium hydroxide as appropriate. The batch was then made up to volume with the water and filtered through a sterile micropore filter into a sterile 10ml amber galls viad (type 1) and sealed with sterile closures and overseals.

Formulation B

| | | |
|---|---|---|
| Active ingredient | | 0.125 g |
| Sterile, pyrogen-free, pH 7 phosphate buffer, | q.s. | to 25ml |

Example 4: Intramuscular injection

| | Weight (g) |
|---|---|
| Active Ingredient | 0.20 |
| Benzyl Alcohol | 0.10 |
| Glycofural 75 | 1.45 |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

Example 5: Syrup

Formulation A

| | Weight (g) |
|---|---|
| Active Ingredient | 0.2500 |
| Sorbitol Solution | 1.5000 |
| Glycerol | 2.0000 |
| Sodium Benzoate | 0.0050 |
| Flavour, Peach 17.42.3169 | 0.0125 ml |
| Purified Water q.s. to | 5.0000 ml |

The active ingredient is dissolved in a mixture of the glycerol and most of the purified water. An aqueous solution of the sodium benzoate is then added to the solution, following by addition of the sorbitol solution and finally the flavour. The volume is made up with purified water and mixed well.

When the active ingredient is poorly soluble, the following formulation (B) is used.

Formulation B

| | Weight (g) |
|---|---|
| Active ingredient | 0.250 |
| Sorbitol Solution | 1.500 |
| Glycerol | 0.005 |
| Dispersible Cellulose | 0.005 |
| Sodium Benzoate | 0.010 ml |
| Flavour | |
| Purified Water to | 5.000 ml |

Mix the sorbitol solution, glycerol and part of the purified water. Dissolve the sodium benzoate in purified water and add the solution to the bulk. Add and disperse the dispersible cellulose and flavour. Add and disperse the active ingredient. Make up to volume with purified water.

Example 6: Suppository

| | mg/suppository |
|---|---|
| Active Ingredient (62µm)* | 250 |
| Hard Fat, BP (Witepsol H15 - Dynamit NoBel | 1770 |
| | 2020 |

* The active ingredient is used as a powder wherein at least 90% of the particles are of 63 µm diameter or less.

One-fifth of the Witepsol H15 is melted in a steam-jacketed pan at 45°C maximum. The active ingredient is sifted through a 200 lm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250 lm stainless steel screen and, with continuous stirring, is allowed to cool to 40°C. At a temperature of 38°C to 40°C, 2.02g of the mixture is filled into suitable, 2ml plastic moulds. The suppositories are allowed to cool to room temperature.

Example 7: Pessaries

| | mg/pessary |
|---|---|
| Active ingredient (63µm) | 250 |
| Anhydrous Dextrose | 380 |
| Potato Starch | 363 |
| Magnesium Stearate | 7 |
| | 1000 |

The above ingredients are mixed directly and pessaries prepared by direct compression of the resulting mixture.
The following Examples 8-10 illustrate the preparation of formulations containing the above Active Ingredient and a further therapeutic nucleoside as specified.

Example 8: Injection

Formulation A

| | Weight (mg) |
|---|---|
| Acyclovir | 400 |
| Active Ingredient | 200 |
| IM NaOH | as required |

(continued)

| | Weight (mg) |
|---|---|
| Sterile Water to | 10 ml |

Formulation B

| | Weight (mg) |
|---|---|
| 2-Amino-9-(2-hydroxyethoxymethyl)purine | 400 |
| Active Ingredient | 200 |
| IM NaOH | as required |
| Sterile Water to | 10 ml |

For the above formulations the therapeutic nucleoside is added to 1M NaOH solution and stirred until dissolved. The active ingredient is added and dissolved. Add sterile water to 10 ml. Filter through a sterile filter and fill into sterile vials. Freeze dry.

For the above formulations the therapeutic nucleoside is added to IM NaOH solution and stirred until dissolved. The active ingredient is added and dissolved. Add Sterile Water to 10ml. Filter through a sterile filter and fill into sterile vials. Freeze dry.

Example 9: Tablets

Formulation A

| | Weight (mg) |
|---|---|
| Acyclovir | 500 |
| Active Ingredient | 125 |
| Povidone | 14 |
| Sodium Starch Glycollate | 25 |
| Magnesium Stearate | 6 |
| | 670 |

Formulation B

| | Weight (mg) |
|---|---|
| Active Ingredient | 125 |
| 2-Amino-9-(2-hydroxyethoxymethyl)purine | 125 |

(continued)

| | Weight (mg) |
|---|---|
| Providone | 8 |
| Sodium Starch Glycollate | 12 |
| Magnesium Stearate | 3 |
| | 273 |

For the above formulations A and B, the therapeutic nucleoside and active igredient are mixed with the sodium starch glycollate and granulated with a solution of povidone. After drying, the granules are blended with magnesium stearate and compressed.

Example 10: Capsules

Formulation A

| | Weight (mg) |
|---|---|
| Acyclovir | 250 |
| Active Ingredient | 62.5 |
| Lactose | 170.5 |
| Sodium Starch Glycollate | 15 |
| Magnesium Stearate | 2 |
| | 500 |

Mix the ingredients and fill into hard gelatin capsules.

Formulation B

| | Weight (mg) |
|---|---|
| Active Ingredient | 125 |
| 2-Amino-9-(2-hydroxyethoxymethyl)purine | 125 |
| Lactose | 133 |
| Sodium Starch Glycollate | 15 |
| Magnesium Stearate | 2 |
| | 400 |

Mix the ingredients and fill into hard gelatin capsules.
Example 11 illustrates a formulation containing interferon and the above Active Ingredient.

Example 11: Injection

| | |
|---|---|
| Interferon | 3 Mega Units |
| Active Ingredient | 200 mg |
| Sterile Buffer pH 7 to | 50 ml |

Dissolve the interferon and active ingredient in the sterile water. Filter through a sterile water filter and fill into sterile vials.

The following Examples 12-15 describe the preparation of formulations containing the above Active Ingredient in combination with a nucleoside transport inhibitor eg dipyridamole.

Example 12: Tablet

| | Weight (mg) |
|---|---|
| Nucleoside transport inhibitor | 300 |
| Active Ingredient | 200 |
| Lactose | 105 |
| Starch | 50 |
| Polyvinylpyrrolidinone | 20 |
| Magnesium stearate | 10 |
| Total weight | 685 |

The active compounds are mixed with the lactose and starch and wet granulated with a solution of the polyvinylpyrrolidinone. The granules are dried, sifted, and blended with magnesium stearate and then compressed into tablet form.

Example 13: Capsule

| | Weight (mg) |
|---|---|
| Nucleoside transport inhibitor | 300 |
| Active Ingredient | 100 |
| Lactose | 100 |
| Sodium starch glycollate | 10 |
| Polyvinylpyrrolidinone | 10 |
| Magnesium stearate | 3 |
| | 523 |

The active compounds are mixed with the lactose and sodium starch glycollate and wet granulated with a solution of the polyvinylpyrro- lidinone. The granules are dried, sifted and blended with the magnesium stearate and filled into hard gelatin capsules.

Examples 14: Cream

| | Weight (g) |
|---|---|
| Nucleoside transport inhibitor | 7.5 |
| Active Ingredient | 5.00 |
| Glycerol | 2.00 |
| Cetostearyl alchohol | 6.75 |
| Sodium lauryl sulphate | 0.75 |
| White soft paraffin | 12.50 |
| Liquid paraffin | 5.00 |
| Chlorocresol | 0.10 |
| Purified Water | to 100.00 |

The active compounds are dissolved in a mixture of purified water and glycerol and heated to 70°C. The remaining ingredients are heated together at 70°C. The two parts are added together and emulsified. The mixture is cooled and filled into containers.

Example 15: Intravenous Injections

| | | Amount (mg) |
|---|---|---|
| (1) | Active Ingredient | 200 |
| | Nucleoside transport inhibitor | 300 |
| | Glycerol | 200 |
| | Sodium hydroxide solution qs | pH 7.0-7.5 |
| | Water for Injections to | 10 ml |

The glycerol is added to some of the water for Injections. The two active compounds are added and the pH adjusted to 7.0-7.5 with sodium hydroxide solution. The solution is made up to volume with additional Water for Injections. Under aseptic conditions, the solution is sterilized by filtration, filled into sterile ampoules and the ampoules and the ampoules sealed.

| | | Amount (mg) |
|---|---|---|
| (2) | Active Ingredient | 100 |
| | Nucleoside transport inhibitor | 150 |
| | Mannitol | 125 |
| | Sodium hydroxide solution qs to | pH 8.0-9.0 |
| | Water for Injections to | 2.5 ml |

The active compounds and the mannitol are dissolved in a part of the water for injections. The pH is adjusted to 8.0-9.0 with the sodium hydroxide solution and made up to volume with additional water for injections. Under aseptic conditions, the solution is sterilized by filtration, filled into sterile vials and the water removed by freeze-drying. The vials are sealed under an atmosphere of nitrogen and closed with a sterile closure and metal collar.

The following Examples 16-18 describe the preparation of formulations containing the Active Ingredient in combination with a glucuronidation\renal excretion inhibitor, for example probenecid.

Example 16: Tablet

| | Weight (mg) |
|---|---|
| Active Ingredient | 100 |
| Glucuronidation/renal excretion inhibitor | 200 |
| Lactose | 105 |
| Starch | 50 |
| Polyvinylpyrrolidinone | 20 |
| Magnesium stearate | 10 |
| Total weight | 485 |

The active compounds are mixed with the lactose and starch and wet granulated with a solution of the polyvinylpyrrolidinone. The granules are dried, sifted, and blended with magnesium stearate and then compressed into tablet form.

Example 17: Capsule

| | Weight (mg) |
|---|---|
| Active Ingredient | 100 |
| Glucuronidation/renal excretion inhibitor | 100 |
| Lactose | 100 |
| Sodium starch glycollate | 10 |
| Polyvinylpyrrolidinone | 10 |

(continued)

| | Weight (mg) |
|---|---|
| Magnesium stearate | 3 |
| Total weight | 323 |

The active compounds are mixed with the lactose and sodium starch glycollate and wet granulated with a solution of the polyvinyl- pyrrolidinone. The granules are dried, sifted and blended with the magnesium stearate and filled into hard gelatin capsules.

Example 18: Intravenous Injections

| | | Amount (mg) |
|---|---|---|
| (1) | Active Ingredient | 200 |
| | Glucuronidation/renal excretion inhibitor | 300 |
| | Glycerol | 200 |
| | Sodium hydroxide solution qs | pH 7.2-7.5 |
| | Water for Injectionsto | 10 ml |

The glycerol is added to some of the water for injections. The two active compounds are added and the pH adjusted to 7.0-7.5 with sodium hydroxide solution. The solution is made up to volume with additional water for injections. Under aseptic conditions, the solution is sterilized by filtration, filled into sterile ampoules and the ampoules sealed.

| | | Amount (mg) |
|---|---|---|
| (2) | Active Ingredient | 100 |
| | Glucuronidation\renal excretion inhibitor | 150 |
| | Mannitol | 125 |
| | Sodium hydroxide solution qs to | pH 8.0-9.0 |
| | Water for Injections | 2.5 ml |

The active compounds and the mannitol are dissolved in a part of the water for injections. The pH is adjusted to 8.0-9.0 with the sodium hydroxide solution and made up to volume with additional water for injections. Under aseptic conditions, the solution is sterilized by filtration, filled into sterile vials and the water removed by freeze- drying. The vials are sealed under an atmosphere of nitrogen and closed with a sterile closure and metal collar.

Example 19: Antiviral Activity

The enhancement of the Anti-Friend Lenkemia Virus (FLV) activity of 3'-azido-3'-deoxythymidine (AZT) by the nucleoside transport inhibitors dipyridamole, dilazep and 6-[(4-nitrobenzyl)thio]-9-b-D-ribofuranosyl) purine is shown in Table 2. FG-10 cells were seeded on a plate one day before they were infected with FLV. One hour after infecting, known concentrations of the test compounds or combinations were added. The plates were incubated for 3 days, the media replaced with fresh McCoy's 5A media and incubated another 3 days. Concentrations of test compound/combinations giving 50% inhibition of plaques were determined as shown in Table 2. Neither dipyridamole (10 µM) nor dilazep (5 µM) alone had any observed antiviral effect.

Table 2

| Compound\Combination | Azidothymidine $ED_{50}$(nM) |
|---|---|
| AZT | 5 |
| AZT + 1 μM dipyridamole | 1 |
| AZT + 5 μM dipyridamole | 0.5 |
| AZT+ 10 μM dipyridamole | 0.2 |
| AZT + 5 μM dilazep | 0.5 |
| AZT + 5 μM 6-[(4-nitrobenzyl)thio]-9-b-D-ribofuranosyl)purine | 3 |

The response of 50% is comparable to that obtained with treatment with the preferred current therapy for KS, recombinant x-interferon.

Example 20: AZT\Acyclovir Combination vs. HIV in Vitro

Using a method analogous to that described in Example 79 of EP21758A, combinations of AZT and acyclovir (ACV) were tested for in vitro efficacy vs. HIV.

ACV alone showed little activity, a concentration of 16 μg/ml (the highest tested) exhibiting less than 30% protection, while AZT at 8μM demonstrated 100% protection by this method.

Table 3

| ACV (lg/ml) | AZT (μM) |
|---|---|
| - | 0 |
| 8 | 0.5 |
| 2 | 1 |
| 1 | 4 |
| 0 | 8 |

These results indicate that ACV potentiates the antiviral activity of AZT about 3-fold.

Example 21: AZT/Interferon Combinations vs. HIV in Vitro

Peripheral blood mononuclear cells (PBMC) from healthy HIV seronegative volunteer donors were obtained by Ficoll-Hypaque sedimentation of heparinized blood. Cells were treated with 10μg/ml of phytohaem- agglutinin (PHA) and grown in medium RPMI 1640 supplemented with 20% fetal calf serum 9FCS0, antibiotics, 1-glutamine and 10% interleukin-2 (IL-2) (Electronucleonics, Bethesda, MD). From 4 to 6 days after exposure to PHA, cells were dispensed at concentrations of 4 x $10^5$ cells/ml in 25 $cm^3$ flasks containing 5 ml medium and then exposed to drugs and virus as detailed below. The day of virus inoculation is referred to as day 0. On day 4 fresh medium was added. Every 3 to 4 days thereafter, a portion of the cell suspension was removed for analysis and replaced with cell-free medium. Experiments 2 and 4 were terminated after 14 days and experiments 1 and 3 after 16 days.

Virus stocks were cell free supernatant fluids of HIV-infected H9 cells frozen in aliquots at -70°C. The 50% tissue culture infectious dose ($TCID_{50}$) of the virus stock was $10^5$/ml.

Four separate experiments were performed using PBMC from different donors (Table 4). In experiment 1, both drugs were added after cells had been exposed to virus. Aliquots of 40 x $10^6$ cells were suspended in 20 ml of medium containing $10^5$ $TCID_{50}$ of virus for 1 hour, then washed 3 times and resuspended in virus free medium. In experiments 2 to 4, cells were incubated for 24 hours in medium with or without recombinant α-interferon (rIFNαA), then exposed to AZT and virus. Virus was added directly to cultures in a small volume of medium and not washed off. The viral inocula were 4 x $10^3$, $10^3$, and 2 x $10^3$ $TCID_{50}$ in experiments 2,3, and 4 respectively. Drug concentrations were adjusted at

each medium change such that original concentrations were maintained.

In all experiments, serial two-fold dilutions of a fixed combination of rIFNA and AZT were studied. Each concentration of of rIFNαA and AZT used in combination was also studied alone to provide points of reference.

In all experiments, duplicate cultures were maintained for each concentration and for infected and uninfected controls. In experiment 1,3.2 µM of AZT and 128 units/ml (U/ml) of rIFNαA, as well as 5 twofold dilutions of this combination, were studied.

In experiments 2 and 3,0.16 lM AZT and 128 U/ml of rIFNαA, and 3-4 twofold dilutions of this combination, were employed. In experiment 4,0.08 µM AZT together with 128 U/ml of rIFNA, and twofold dilutions of this combination, were employed.

After approximately one week, cultures were evaluated every 3 to 4 days for presence of virus. Cells were evaluated for HIV antigens by indirect immunofluorescence; the supernatant fluids were evaluated for reverse transcriptase (RT) activity, virus yield, and for HIV p24 antigen by radioimmunoassay.

The multiple drug effect analysis of Chou and Talalay (Advances in Enzyme Regulation, (1984), 22, 27-55) was used to calculate combined drug effects.

Data was also evaluated by the isobologram technique, a geometric method for assessing drug interactions. The concentration of AZT producing a desired (e.g. 50% inhibitory) effect is plotted on the horizontal axis, and the concentration of rIFNαA producing the same degree of effect is plotted on the vertical axis. A line is drawn connecting these points and the concentration of the agents in combination which produces the same effect is plotted. If this point falls below the line, the combination is considered synergistic.

In experiment 1, all concentrations of AZT were fully inhibitory, making it impossible to judge combined effects. In experiments 2-4, a synergistic interaction of the two agents was consistently observed (Tables 4-9). The synergism was evident by all measures of viral replication emplyed, and persisted even when the effect of rIFNαA alone was negligible. Synergy calculations were performed by applying the multiple drug effect analysis to RT data from experiments 2-4, virus yield data from experiment 2 and 3, and RIA data from experiment 4. The isobologram method also indicated synergism.

Table 4

| HIV Inoculation | | HIV added ($TCID_{50}$) | Timing of Drug Addition | |
|---|---|---|---|---|
| Experiment Method[a] | | | rIFNαA | AZT |
| 1 | A | $10^5$ | 0 | 0 |
| 2 | B | $4x10^3$ | -24hours | 0 |
| 3 | B | $10^3$ | -24 hours | 0 |
| 4 | B | $2 \times 10^3$ | -24 hours | 0 |

(a) method A: $40 \times 10^6$ cells were suspended in 20 ml of medium containing $10^5$ $TCID_{50}$ HIV for 1 hour at 37°C, then washed and resuspended.
method B: Indicated amount of virus was added to $2 \times 10^6$ cells in medium; cells not subsequently washed.

Table 5

| Experiment 2 - Day 10<br>Effects of rIFNαA and AZT on mean reverse transcriptase values<br>($cpm/10^6$ cells $\times 10^3$)<br>rIFNA (U/ml) | | | | | | |
|---|---|---|---|---|---|---|
| AZT(µM) | 0 | 8 | 16 | 32 | 64 | 128 |
| 0 | 205 | 173 | 150 | 193 | 169 | 151 |
| 0.01 | 159 | 85 | | | | |
| 0.02 | 110 | | 42 | | | |
| 0.04 | 71 | | | 10 | | |

Table 5 (continued)

| Experiment 2 - Day 10<br>Effects of rIFNαA and AZT on mean reverse transcriptase values ($cpm/10^6$ cells x $10^3$)<br>rIFNA (U/ml) | | | | | | |
|---|---|---|---|---|---|---|
| AZT(μM) | 0 | 8 | 16 | 32 | 64 | 128 |
| 0.08 | 31 | | | | 4 | |
| 0.16 | 7 | | | | | 1 |

Table 6

| Experiment 3 - Day 13<br>Effects of rIFNαA and AZT on mean RT values ($cpm/10^6$ cells x $10^3$)<br>rIFNA (U/ml) | | | | | |
|---|---|---|---|---|---|
| AZT(μM) | 0 | 16 | 32 | 64 | 128 |
| 0 | 157 | 143 | 117 | 117 | 130 |
| 0.02 | 51 | 10 | | | |
| 0.04 | 10 | | 0 | | |
| 0.08 | 2 | | | 0 | |
| 0.16 | 5 | | | | 0 |

Table 7

| Experiment 4 - Day 11<br>Effects of rIFNαA and AZT on mean RT values ($cpm/10^6$ cells x $10^3$)<br>rIFNαA (U/ml) | | | | |
|---|---|---|---|---|
| AZT(IM) | 0 | 32 | 64 | 128 |
| 0 | 32 | 5 | 4 | 2 |
| 0.02 | 8 | 1 | | |
| 0.04 | 4 | | 0 | |
| 0.08 | 1 | | | 0 |

Table 8

| Experiment 3 - Day 13<br>Effects of rIFNαA and AZT on virus yield ($TCID_{50}$ /ml).<br>rIFNαA (U/ml) | | | | | |
|---|---|---|---|---|---|
| AZT(IM) | 0 | 16 | 32 | 64 | 128 |
| 0 | $10^{5.7}$ | $10^{5.6}$ | $10^{5.3}$ | $10^{5.1}$ | $10^{5.0}$ |

Table 8 (continued)

| Experiment 3 - Day 13<br>Effects of rIFNαA and AZT on virus yield ($TCID_{50}$ /ml).<br>rIFNαA (U/ml) | | | | | |
|---|---|---|---|---|---|
| AZT(IM) | 0 | 16 | 32 | 64 | 128 |
| .02 | $10^{4.8}$ | $10^{1.9}$ | | | |
| .04 | $10^{3}$ | | $<10^{1.9}$ | | |
| .08 | $10^{1.9}$ | | | $<10^{1.9}$ | |
| .16 | $<10^{1.9}$ | | | | $<10^{1.9}$ |

Table 9

| Experiment 4 - Day 14<br>Effects of rIFNαA and AZT on HIV p24[a]<br>IFNA (U/ml) | | | | |
|---|---|---|---|---|
| AZT(IM) | 0 | 32 | 64 | 128 |
| 0 | 200-300 | 100-200 | 50-100 | 25-30 |
| 0.02 | 100-200 | 2.2 | | |
| 0.04 | 25-30 | | 1.0 | |
| 0.08 | 11.2 | | | 0.8 |

a HIV p24 levels are presented as ng protein/ml

Table 10

| Combination Indices for AZT and rIFNαA Calculated from RT data.<br>Combination Indices at Different Percentages of RT Inhibition | | | | |
|---|---|---|---|---|
| Day in | | | | |
| Experiment | Culture | 50% | 90% | 95% |
| 2 | 7 | 2.14 | 0.34 | 0.23 |
| 2 | 10 | 0.37 | 0.30 | 0.28 |
| 3 | 6 | 0.26 | 0.73 | 1.39 |
| 3 | 13 | 0.12 | 0.15 | 0.17 |
| 3 | 16 | <0.01 | 0.02 | 0.05 |
| 4 | 8 | 0.01 | 0.03 | 0.04 |
| 4 | 14 | 0.02 | 0.07 | 0.12 |

C.I. values are determined by solving the equation for different degrees of RT inhibition. C.I. values <1 indicates synergism. The C.I. values given were obtained using the mutually non-exclusive form of the equation; values obtained using the mutually exclusive form were always slightly lower.

Example 22: In Vitro Antibacterial Synergy Studies

3'-Azido-3'-deoxythymidine and 8 known antibacterial agents (listed in Table 11) were each treated with N,N-dimethylformamide for 30 minutes. Microtiter dilutions were prepared using Wellcotest broth.
Prior to synergy testing, MIC determinations were made for each compound individually against the test organism (E. coli CN314). Table 11 shows the MIC endpoints of each drug for E. coli CN314.
Two-fold serial dilutions of test drugs or 3'-azido-3'-deoxythymidine were prepared in "flat-bottom" or "transfer" microtiter plates, respectively. Plates containing the appropriate dilutions were combined, resulting in a series of 192 dilutions/combination. Controls consisting of compound alone were also included. The highest concentration of any drug used, was twice its MIC value (Table 11).
Test plates were inoculated with a bacterial seed culture containing approximately 5 x $10^5$ CFU/ml and were subsequently incubated at 27°C for 18 hours. The wells were scored for bacterial growth or no growth, and the MICs determined. "Fractional inhibitory concentrations" (FICs) were calculated from MIC values by dividing the MIC in combination by the MID of each single agent. The sum of the fractions (sum of the fractional inhibitory concentrations) was then calculated. A result of about 0.5, or less, is indicative of synergy.

Table 11

| Minimal Inhibitory Concentrations (MIC's) of Test Drugs Alone | |
|---|---|
| Compound | MIC (lg/ml) |
| Tobramycin | 0.4 |
| Fusidic acid | 1000 |
| Chloramphenicol | 3.1 |
| Clindamycin | 100 |
| Erthromycin | 25 |
| Rifampicin | 6.2 |
| 3'-Azido-3'-deoxythymidine | 1.0 |
| Trimethoprim | 0.125 |
| Sulphadimidine | 32 |

Results of the synergy experiments are shown in Table 12

Table 12

| Synergy Studies: Combinations of AZT and Other Antibacterial Agents | | |
|---|---|---|
| Combination Index | Optimal MIC's (1g/ml): | |
| | Drug/AZT | FIC |
| Tobramycin/AZT | 0.2/0.125 | 0.25 |
| Fusidic acid/AZT | 250/0.03 | 0.28 |
| Chloramphenicol/AZT | 1.6/0.06 | 0.31 |
| Clindamycin/AZT | 12.5/0.25 | 0.375 |
| Erythromycin\AZT | 6.2/0.25 | 0.5 |
| Rifampicin/AZT | 3.1/0.06 | 0.56 |
| Trimethoprim/AZT | 0.004/0.5 | 0.504 |
| Sulfadimidine/AZT | 0.25/0.125 | 0.375 |

## Claims

1. A therapeutic combination of 3'-azido-3'-deoxythymidine or a pharmaceutically acceptable derivative thereof and at least one further therapeutic agent selected from a glucuronidation inhibitor and/or a renal excretion inhibitor, a nucleoside transport inhibitor, a further therapeutic nucleoside, an antibacterial agent, an interferon, interleukin II, suramin, phosphonoformate, HPA 23 and a 2',3'-dideoxynucleoside.

2. A combination according to claim 1 wherein the second agent is a glucuronidation inhibitor and/or renal excretion inhibitor selected from probenecid, aspirin, acetaminophen, lorazepam, cimetidine, ranitidine, zomepirac, clofibrate, indomethacin, ketoprofen and naproxen.

3. A combination according to claim 1 wherein the second agent is a nucleotides transport inhibitor selected from dilazep, dipyridamole, 6-[(4-nitrobenzoyl)thio]-9-(b-D-ribofuranosyl) purine, papavarine, mioflazine, hexobendine and lidoflazine and acid addition salts thereof.

4. A combination according to claim 1 wherein the second agent is a further therapeutic nucleoside selected from 9-[(2-hydroxy-1-hydroxymethylthoxy)methyl]guanine, 2-amino-9-(2-hydroxyethoxy- methyl)purine and 9-(2-hydroxythoxymethyl)guanine.

5. A combination according to claim 1 wherein the second agent is a 2',3'-dideoxynucleoside selected from 2',3'-dideoxycytidine and 2',3'-dideoxyadenosine.

6. A combination according to claim 1 wherein the second agent is an antibacterial agent selected from 2,4-diamino-5-(3',4',5'-triomethoxybenzyl)pyrimidine or an analogue thereof, sulfadimidine, rifampicin, tobramycin, fusidic acid, chloramphenicol, clindamycin and erythromycin.

7. A combination according to claim 1 wherein the second agent is 9-(2-hydroxythoxymethyl)guanine.

8. A combination according to any of the preceding claims for use in the treatment or prophylaxis of a human retroviral infection.

9. A combination according to claim 8 wherein the said infection is an HTLV infection.

10. A combination according to claim 9 wherein the said HTLV infection is an HTLV-I or HTLV-II infection.

11. A combination according to claim 8 wherein the said infection is an HIV infection.

12. A combination according to any of claims 1 to 7 for use in the treatment or prophylaxis of AIDS.

13. A combination according to any of claims 1 to 7 for use in the treatment or prophylaxis of AIDS-related complex.

14. A combination according to any of claims 1 to 7 for use in the treatment or prophylaxis of persistent generalised lymphadenopathy.

15. A combination according to any of claims 1 to 7 for use in the treatment or prophylaxis of the asymptomatic AIDS-carrier state.

## Patentansprüche

1. Therapeutische Kombination aus 3'-Azido-3'-desoxythymidin oder einem pharmazeutisch akzeptablen Derivat davon und wenigstens einem weiteren Therapeutikum, ausgewählt aus einem Glucuronidierungs-Inhibitor und/oder einem Nierenexkretions-Inhibitor, einem Nukleosid-Transportinhibitor, einem weiteren therapeutischen Nukleosid, einem bakteriziden Mittel, einem Interferon, Interleukin II, Suramin, Phosphonoformiat, HPA 23 und einem 2',3'-Didesoxynukleosid.

2. Kombination gemäß Anspruch 1, worin das zweite Mittel ein Glucuronidierungs-Inhibitor und/oder Nierenexkretions-Inhibitor ist, ausgewählt aus Probenecid, Aspirin, Acetaminophen, Lorazepam, Cimetidin, Ranitidin, Zomepirac, Clofibrat, Indomethacin, Ketoprofen und Naproxen.

3. Kombination gemäß Anspruch 1, worin das zweite Mittel ein Nukleosid-Transportinhibitor ist, ausgewählt aus Dilazep, Dipyridamol, 6-[(4-Nitrobenzoyl)thio]-9-(b-D-ribofuranosyl)purin, Papavarin, Mioflazin, Hexobendin und Lidoflazin und Säureadditionssalzen davon.

4. Kombination gemäß Anspruch 1, worin das zweite Mittel ein weiteres therapeutisches Nukleosid ist, ausgewählt aus 9-[(2-Hydroxy-1-hydroxymethylethoxy)methyl]guanin, 2-Amino-9-(2-hydroxyethoxymethyl)purin und 9-(2-Hydroxyethoxymethyl)guanin.

5. Kombination gemäß Anspruch 1, worin das zweite Mittel ein 2'3'-Didesoxynukleosid ist, ausgewählt aus 2',3'-Didesoxycytidin und 2',3'-Didesoxyadenosin.

6. Kombination gemäß Anspruch 1, worin das zweite Mittel ein bakterizides Mittel ist, ausgewählt aus 2,4-Diamino-5-(3',4',5'-triomethoxybenzyl)pyrimidin oder einem Analogon davon, Sulfadimidin, Rifampicin, Tobramycin, Fusidinsäure, Chloramphenicol, Clindamycin und Erythromycin.

7. Kombination gemäß Anspruch 1, worin das zweite Mittel 9-(2-Hydroxyethoxymethyl)guanin ist.

8. Kombination gemäß einem der vorhergehenden Ansprüche zur Verwendung in der Behandlung von Prophylaxe einer menschlichen Retrovirusinfektion.

9. Kombination gemäß Anspruch 8, worin die Infektion eine HTLV-Infektion ist.

10. Kombination gemäß Anspruch 9, worin die HTLV-Infektion eine HTLV-I- oder HTLV-II-Infektion ist.

11. Kombination gemäß Anspruch 8, worin die Infektion eine HIV-Infektion ist.

12. Kombination gemäß einem der Ansprüche 1 bis 7 zur Verwendung in der Behandlung oder Prophylaxe von AIDS.

13. Kombination gemäß einem der Ansprüche 1 bis 7 zur Verwendung in der Behandlung oder Prophylaxe des AIDS-bezogenen Komplexes ("AIDS-related complex").

14. Kombination gemäß einem der Ansprüche 1 bis 7 zur Verwendung in der Behandlung oder Prophylaxe von anhaltender allgemeiner Lymphadenopathie.

15. Kombination gemäß einem der Ansprüche 1 bis 7 zur Verwendung in der Behandlung oder Prophylaxe des asymptomatischen AIDS-Trägerzustands ("AIDS-carrier state").

**Revendications**

1. Combinaison thérapeutique de 3'-azido-3'-désoxythymidine ou d'un dérivé pharmaceutiquement acceptable de celle-ci et d'au moins un autre agent thérapeutique choisi parmi un inhibiteur de glucuronidation et/ou un inhibiteur d'excrétion rénale, un inhibiteur de transport de nucléoside, un autre nucléoside thérapeutique, un agent antibactérien, un interféron, l'interleukine II, la suramine, le phosphonoformiate, l'HPA 23 et un 2',3'-didésoxynucléoside.

2. Combinaison suivant la revendication 1, dans laquelle le second agent est un inhibiteur de glucuronidation et/ou un inhibiteur d'excrétion rénale choisi parmi le probénécid, l'aspirine, l'acétaminophène, le lorazépam, la cimétidine, la ranitidine, le zomépirac, le clofibrate, l'indométhacine, le cétoprofène et le naproxène.

3. Combinaison suivant la revendication 1, dans laquelle le second agent est un inhibiteur de transport de nucléoside choisi parmi le dilazep, le dipyridamole, la 6-[(4-nitrobenzoyl)thio]-9-(b-D-ribofurannosyl)purine, la papavarine, la mioflazine, l'hexobendine et la lidoflazine et leurs sels d'addition d'acide.

4. Combinaison suivant la revendication 1, dans laquelle le second agent est un autre nucléoside thérapeutique choisi parmi la 9-[(2-hydroxy-1-hydroxyméthylethoxy)méthyl]guanine, la 2-amino-9-(2-hydroxyéthoxyméthyl)purine et la 9-(2-hydroxyéthoxyméthyl)guanine.

5. Combinaison suivant la revendication 1, dans laquelle le second agent est un 2',3'-didésoxynucléoside choisi parmi la 2',3'-didésoxycitidine et la 2',3'-didésoxyadénosine.

6. Combinaison suivant la revendication 1, dans laquelle le second agent est un agent antibactérien choisi parmi la 2,4-diamino-5-(3',4',5'-triméthoxybenzyl)pyrimidine ou un analogue de celle-ci, la sulfadimidine, la rifampicine, la tobramycine, l'acide fusidique, le chloramphénicol, la clindamycine et l'érythromycine.

7. Combinaison suivant la revendication 1, dans laquelle le second agent est la 9-(2-hydroxyéthoxyméthyl)guanine.

8. Combinaison suivant l'une quelconque des revendications précédentes utilisable dans le traitement ou la prophylaxie d'une infection rétrovirale humaine.

9. Combinaison suivant la revendication 8, dans laquelle ladite infection est une infection au HTLV.

10. Combinaison suivant la revendication 9, dans laquelle ladite infection au HTLV est une infection au HTLV-I ou HTLV-II.

11. Combinaison suivant la revendication 8, dans laquelle ladite infection est une infection au VIH.

12. Combinaison suivant l'une quelconque des revendications 1 à 7 utilisable dans le traitement ou la prophylaxie du sida.

13. Combinaison suivant l'une quelconque des revendications 1 à 8 utilisable dans le traitement ou la prophylaxie du complexe associé au sida.

14. Combinaison suivant l'une quelconque des revendications 1 à 7 utilisable dans le traitement ou la prophylaxie de la lymphadénopathie généralisée persistante.

15. Combinaison suivant l'une quelconque des revendications 1 à 7 utilisable dans le traitement ou la prophylaxie de l'état porteur du sida asymptomatique.